# EUROPEAN PATENT APPLICATION

(11) **EP 4 534 086 A1**
(43) Date of publication of application: **09.04.2025**
(21) Application number: 23386091.5
(22) Date of filing: 06.10.2023
(51) Int. Cl.: A61K 31/445, A61P 25/28

(54) **TREATMENT OF LEUKODYSTROPHY**

(71) Applicant: The Provost, Fellows, Foundation Scholars, & the other members of Board, of the College of the Holy & Undiv. Trinity of Queen Elizabeth near Dublin, Dublin 2 (IE); National and Kapodistrian University of Athens, 10561 Athens (GR)
(72) Inventor: Dev, Kumlesh, Dublin 2 (IE); Valsami, Georgia, 15784 Athens (GR); Papakyriakopoulou, Paraskevi, 15784 Athens (GR)
(74) Representative: Yazitzoglou, Evagelia S.

(57) **Abstract**

The present invention relates to the treatment of leukodystrophy. Specifically, the present invention relates to the treatment of leukodystrophies such as globoid cell leukodystrophy using acetylcholinesterase inhibitors such as donepezil. Also disclosed are methods of treatment of leukodystrophies such as globoid cell leukodystrophy and uses of acetylcholinesterase inhibitors such as donepezil in the treatment of leukodystrophies such as globoid cell leukodystrophy. Also disclosed is a method for diagnosing, detecting or identifying a leukodystrophy in a subject.

## Description

### Field of the invention

The present invention relates to the treatment of leukodystrophy. Specifically, the present invention relates to the treatment of leukodystrophies such as globoid cell leukodystrophy using acetylcholinesterase inhibitors such as donepezil.

### Background to the invention

Alzheimer's disease (AD) is characterized by progressive cognitive decline and histopathological hallmarks including neurofibrillary tangles and plaques composed of post-transcriptional hyperphosphorylated forms of the tau microtubule-associated protein and the proteolytic amyloid-beta (Aβ) peptide derivatives of the amyloid precursor protein (APP), respectively. Disease-modifying therapeutic strategies for AD have focused primarily on targeting both these disease-related hallmarks of amyloid-beta accumulation and tau hyperphosphorylation. Cellular and mechanistic events associated with AD and progression of the disease additionally proposed as drug targets include neuroinflammation and glial cell hypertrophy. While not afforded a central position of attention, aberrations in myelin, firstly reported in the originating findings by Alois Alzheimer in 1911, are also associated with this neurodegenerative disease. Indeed, it is recognized that impairment of oligodendrocyte function appears early in AD and is related to the white matter disruption, occurring before the amyloid-beta accumulation. The reverse sequence of events has also been proposed, where amyloid-beta has a detrimental effect on oligodendrocyte maturation resulting in amyloid-beta-mediated demyelination. Despite being somewhat adjacent, evidence gathered on the currently marketed therapeutic strategies for AD, namely acetylcholinesterase inhibitors (AChEi), also regulate myelin state that may be part of a mechanism related to the clinical efficacy of these drugs.

AChEis impede the hydrolysis of acetylcholine (ACh), increase the levels of this neurotransmitter in the synaptic cleft and thereby enhance cholinergic transmission. Cholinergic activation is considered critical for the maintenance of neuronal function and ensuring successful synaptic transmission in peripheral and central nervous systems. AchEi such as donepezil, rivastigmine, and tacrine improve cognitive function and are used for the symptomatic management of AD. Marketed AChEi drugs have been reported to have potential disease-modifying targeting mechanisms. For example, these drugs appear to delay the deposition of amyloid plaques and attenuate microglia activation. AChE inhibition is also related to reduced lymphocyte proliferation. Cholinergic neurotransmission also plays a role in regulating the secretion of proinflammatory cytokines (such as TNF-alpha, IL-6 and IL-1-beta). Drugs such as donepezil and rivastigmine are also suggested to play a role in regulating myelin state.

The effect of donepezil on demyelination has been assessed in cellular studies such as on oligodendrocyte precursor cells (OPCs) seeded alone and in co-culture with dorsal root ganglion (OPC-DRG) neurons. The drug managed to increase the length of myelinated axons and promote OPC differentiation to oligodendrocytes (OLs). Moreover, the number of myelinated axons, as well as the G-ratio of remyelinated axons in the corpus callosum region of mice were found to have increased in further in vivo experiments. Donepezil also upregulates the expression of myelin-related genes in OPCs. AchEi drugs such as donepezil and rivastigmine are also agonists for sigma-1-receptors (Sig-1R) and bind to these receptors at a low-nanomolar in range similar to AChE. Sufficient evidence now exists to argue that efficacy of some AChEi drugs may be via a dual AchE inhibition and Sig-1R agonist mechanism. Notably, Sig-1R agonists have been suggested as potential use in demyelination diseases such as multiple sclerosis and vanishing white matter (VWM) disease.

Globoid cell leukodystrophy is a demyelinating disease associated with oligodendrocyte cell death and aberrant myelin state. Globoid cell leukodystrophy, also known as Krabbe disease (KD), is a rare autosomal recessive disease occurring in approximately 1:100,000. This disease is a lipid storage disorder caused by mutations in the *galc* gene, which codes for the enzyme galactosylceramidase (GALC). Mutations in *galc* result in a loss of function and the toxic build-up of metabolites galactosylceramidase and galactosylsphingosine (psychosine). Psychosine is known to be toxic to oligodendrocytes and causes demyelination by various proposed mechanisms, where myelin loss and neuroinflammation are presumably triggered by consequences of absent GALC function not clearing psychosine. Globoid cell leukodystrophy is also associated with a cluster of glial and immune cell dysfunctions including astrogliosis, microglia activation, as well as the macrophage recruitment. A range of mechanisms that attenuate psychosine-induced cell toxicity and demyelination have been previously described, including (i) sphingosine 1-phosphate receptor functional antagonists, (ii) phospholipase A2 inhibitors, (iii) piezo 1 mechanosensitive ion channel antagonists, (iv) hybrid nanoparticles and (v) D2 receptor antagonists antipsychotics. In particular, marketed drugs fingolimod and haloperidol have been described to promote lifespan in the twitcher mouse model of KD.

There is a need to provide new means for the treatment of leukodystrophies such as globoid cell leukodystrophy.

### Summary of the invention

According to a first aspect of the present invention there is provided an acetylcholinesterase inhibitor for use in the treatment of a leukodystrophy.

Optionally, the use comprises administering a therapeutically effective amount of the acetylcholinesterase inhibitor to a subject.

According to a second aspect of the present invention there is provided a method for the treatment of a leukodystrophy in a subject in need thereof, the method comprising administering a therapeutically effective amount of an acetylcholinesterase inhibitor to the subject, thereby treating the leukodystrophy.

According to a third aspect of the present invention there is provided use of an acetylcholinesterase inhibitor in the manufacture of a medicament for the treatment of a leukodystrophy.

According to a fourth aspect of the present invention there is provided use of an acetylcholinesterase inhibitor in the treatment of a leukodystrophy.

Alternatively or additionally, there is provided a sigma-1 receptor (Sig-1R) agonist for use in the treatment of a leukodystrophy, optionally wherein the use comprises administering a therapeutically effective amount of the sigma-1 receptor (Sig-1R) agonist to a subject.

Alternatively or additionally, there is provided a method for the treatment of a leukodystrophy in a subject in need thereof, the method comprising administering a therapeutically effective amount of a sigma-1 receptor (Sig-1R) agonist to the subject, thereby treating the leukodystrophy.

Alternatively or additionally, there is provided use of a sigma-1 receptor (Sig-1R) agonist in the manufacture of a medicament for use in the treatment of a leukodystrophy.

Alternatively or additionally, there is provided use of a sigma-1 receptor (Sig-1R) agonist in the treatment of a leukodystrophy.

Optionally, the leukodystrophy is globoid cell leukodystrophy.

Preferably, the leukodystrophy is globoid cell leukodystrophy.

Optionally, the acetylcholinesterase inhibitor and/or the sigma-1 receptor (Sig-1R) agonist has acetylcholinesterase inhibitor and/or sigma-1 receptor (Sig-1R) agonist activity.

Further optionally, the acetylcholinesterase inhibitor and/or the sigma-1 receptor (Sig-1R) agonist has dual acetylcholinesterase inhibitor and sigma-1 receptor (Sig-1R) agonist activity.

Optionally, the acetylcholinesterase inhibitor is a reversible acetylcholinesterase inhibitor.

Optionally, the sigma-1 receptor (Sig-1R) agonist is a reversible sigma-1 receptor (Sig-1R) agonist.

Optionally, the acetylcholinesterase inhibitor and/or the sigma-1 receptor (Sig-1R) agonist is donepezil ((RS)-2-[(1-Benzyl-4-piperidyl)methyl]-5,6-dimethoxy-2,3-dihydroinden-1-one).

Optionally, the acetylcholinesterase inhibitor and/or the sigma-1 receptor (Sig-1R) agonist is donepezil hydrochloride.

Preferably, the acetylcholinesterase inhibitor and/or the sigma-1 receptor (Sig-1R) agonist is donepezil.

Optionally, the acetylcholinesterase inhibitor and/or the sigma-1 receptor (Sig-1R) agonist is administered orally and/or topically.

Optionally, the acetylcholinesterase inhibitor and/or the sigma-1 receptor (Sig-1R) agonist is administered orally and/or transdermally.

Preferably, the acetylcholinesterase inhibitor and/or the sigma-1 receptor (Sig-1R) agonist is administered orally.

Optionally, the acetylcholinesterase inhibitor and/or the sigma-1 receptor (Sig-1R) agonist is administered at a dose of about 1-20, optionally about 5-15, optionally about 10 mg/kg.

Optionally, the acetylcholinesterase inhibitor and/or the sigma-1 receptor (Sig-1R) agonist is administered at a dose of about 1-20, optionally about 5-15, optionally about 10mg/kg per day.

Preferably, the acetylcholinesterase inhibitor and/or the sigma-1 receptor (Sig-1R) agonist is administered at a dose of about 10mg/kg per day.

Optionally, the acetylcholinesterase inhibitor and/or the sigma-1 receptor (Sig-1R) agonist is administered for about 1-80, optionally about 10-70, optionally about 20-60, optionally about 30-50, optionally about 40-50, optionally about 44 days.

Preferably, the acetylcholinesterase inhibitor and/or the sigma-1 receptor (Sig-1R) agonist is administered for about 44 days.

Optionally, administration of the acetylcholinesterase inhibitor and/or the sigma-1 receptor (Sig-1R) agonist increases a level of expression of myelin basic protein (MBP).

Further optionally, administration of the acetylcholinesterase inhibitor and/or the sigma-1 receptor (Sig-1R) agonist increases a level of expression of myelin basic protein (MBP) relative to a level of expression of myelin basic protein (MBP) prior to administration.

Optionally, administration of the acetylcholinesterase inhibitor and/or the sigma-1 receptor (Sig-1R) agonist decreases an amount of vimentin in a white matter (WM) of the cerebellar cortex.

Further optionally, administration of the acetylcholinesterase inhibitor and/or the sigma-1 receptor (Sig-1R) agonist decreases an amount of vimentin in a white matter (WM) of the cerebellar cortex prior to administration.

Optionally, administration of the acetylcholinesterase inhibitor and/or the sigma-1 receptor (Sig-1R) agonist decreases an amount of vimentin in a white matter (WM) of the cerebellar cortex relative to a granular layer (GL) of the cerebellar cortex.

Further optionally, administration of the acetylcholinesterase inhibitor and/or the sigma-1 receptor (Sig-1R) agonist decreases an amount of vimentin in a white matter (WM) of the cerebellar cortex relative to a granular layer (GL) of the cerebellar cortex prior to administration.

Optionally, administration of the acetylcholinesterase inhibitor and/or the sigma-1 receptor (Sig-1R) agonist decreases a level of expression of ionized calcium binding adaptor molecule 1 (lba1).

Further optionally, administration of the acetylcholinesterase inhibitor and/or the sigma-1 receptor (Sig-1R) agonist decreases a level of expression of ionized calcium binding adaptor molecule 1 (lba1) relative to a level of expression of Iba1 prior to administration.

Optionally, administration of the acetylcholinesterase inhibitor and/or the sigma-1 receptor (Sig-1R) agonist decreases a level of expression of at least one of vimentin, ionized calcium binding adaptor molecule 1 (lba1), and interleukin-6 (IL-6) relative to the level of expression of at least one of myelin basic protein (MBP), and myelin oligodendrocyte glycoprotein (MOG).

Further optionally, administration of the acetylcholinesterase inhibitor and/or the sigma-1 receptor (Sig-1R) agonist decreases a level of expression of at least one of vimentin, ionized calcium binding adaptor molecule 1 (Iba1), and interleukin-6 (IL-6) relative to the level of expression of at least one of myelin basic protein (MBP), and myelin oligodendrocyte glycoprotein (MOG) prior to administration.

Optionally, administration of the acetylcholinesterase inhibitor and/or the sigma-1 receptor (Sig-1R) agonist decreases a level of expression of vimentin, ionized calcium binding adaptor molecule 1 (lba1), and interleukin-6 (IL-6) relative to the level of expression of myelin basic protein (MBP), and myelin oligodendrocyte glycoprotein (MOG).

Further optionally, administration of the acetylcholinesterase inhibitor and/or the sigma-1 receptor (Sig-1R) agonist decreases a level of expression of vimentin, ionized calcium binding adaptor molecule 1 (lba1), and interleukin-6 (IL-6) relative to the level of expression of myelin basic protein (MBP), and myelin oligodendrocyte glycoprotein (MOG) prior to administration.

Optionally, administration of the acetylcholinesterase inhibitor and/or the sigma-1 receptor (Sig-1R) agonist decreases a level of expression of vimentin, and ionized calcium binding adaptor molecule 1 (lba1) relative to the level of expression of myelin basic protein (MBP), and myelin oligodendrocyte glycoprotein (MOG).

Further optionally, administration of the acetylcholinesterase inhibitor and/or the sigma-1 receptor (Sig-1R) agonist decreases a level of expression of vimentin, and ionized calcium binding adaptor molecule 1 (Iba1) relative to the level of expression of myelin basic protein (MBP), and myelin oligodendrocyte glycoprotein (MOG) prior to administration.

Optionally, administration of the acetylcholinesterase inhibitor and/or the sigma-1 receptor (Sig-1R) agonist increases body weight.

Further optionally, administration of the acetylcholinesterase inhibitor and/or the sigma-1 receptor (Sig-1R) agonist increases body weight relative to body weight prior to administration.

Optionally, administration of the acetylcholinesterase inhibitor and/or the sigma-1 receptor (Sig-1R) agonist increases the lifespan of the subject.

Optionally, administration of the acetylcholinesterase inhibitor and/or the sigma-1 receptor (Sig-1R) agonist increases the lifespan of the subject by about 25% relative to the lifespan of a subject suffering from a leukodystrophy.

According to a further aspect of the present invention there is provided a method for diagnosing, detecting or identifying a leukodystrophy in a subject, the method comprising determining a level of expression of myelin basic protein (MBP) in the subject, wherein a level of expression of myelin basic protein (MBP) in the subject less than a level of expression of myelin basic protein (MBP) in a subject not suffering from a leukodystrophy indicates a leukodystrophy in the subject.

Alternatively or additionally, there is provided a method for diagnosing, detecting or identifying a leukodystrophy in a subject, the method comprising determining a level of expression of myelin oligodendrocyte glycoprotein (MOG) in the subject, wherein a level of expression of myelin oligodendrocyte glycoprotein (MOG) in the subject less than a level of expression of myelin oligodendrocyte glycoprotein (MOG) in a subject not suffering from a leukodystrophy indicates a leukodystrophy in the subject.

Alternatively or additionally, there is provided a method for diagnosing, detecting or identifying a leukodystrophy in a subject, the method comprising determining a level of expression of vimentin in the subject, wherein a level of expression of vimentin in the subject greater than a level of expression of vimentin in a subject not suffering from a leukodystrophy indicates a leukodystrophy in the subject.

Alternatively or additionally, there is provided a method for diagnosing, detecting or identifying a leukodystrophy in a subject, the method comprising determining a level of expression of ionized calcium binding adaptor molecule 1 (Iba1) in the subject, wherein a level of expression of ionized calcium binding adaptor molecule 1 (Iba1) in the subject greater than a level of expression of ionized calcium binding adaptor molecule 1 (lba1) in a subject not suffering from a leukodystrophy indicates a leukodystrophy in the subject.

Alternatively or additionally, there is provided a method for diagnosing, detecting or identifying a leukodystrophy in a subject, the method comprising determining a level of expression of interleukin-6 (IL-6) in the subject, wherein a level of expression of interleukin-6 (IL-6) in the subject greater than a level of expression of interleukin-6 (IL-6) in a subject not suffering from a leukodystrophy indicates a leukodystrophy in the subject.

Optionally, the method comprises the step of comparing the level of expression of at least one of myelin basic protein (MBP), myelin oligodendrocyte glycoprotein (MOG), vimentin, ionized calcium binding adaptor molecule 1 (lba1), and interleukin-6 (IL-6) in the subject with the level of expression of at least one of myelin basic protein (MBP), myelin oligodendrocyte glycoprotein (MOG), vimentin, ionized calcium binding adaptor molecule 1 (lba1), and interleukin-6 (IL-6) in the subject.

Further optionally, the method comprises the step of comparing the level of expression of at least one of vimentin, ionized calcium binding adaptor molecule 1 (lba1), and interleukin-6 (IL-6) in the subject with the level of expression of at least one of myelin basic protein (MBP), and myelin oligodendrocyte glycoprotein (MOG) in the subject.

Still further optionally, the method comprises the step of comparing the level of expression of vimentin, ionized calcium binding adaptor molecule 1 (Iba1), and interleukin-6 (IL-6) in the subject with the level of expression of myelin basic protein (MBP), and myelin oligodendrocyte glycoprotein (MOG) in the subject.

Still further optionally, the method comprises the step of comparing the level of expression of at least one of vimentin, and ionized calcium binding adaptor molecule 1 (lba1) in the subject with the level of expression of at least one of myelin basic protein (MBP), and myelin oligodendrocyte glycoprotein (MOG) in the subject.

Still further optionally, the method comprises the step of comparing the level of expression of vimentin, and ionized calcium binding adaptor molecule 1 (that) in the subject with the level of expression of myelin basic protein (MBP), and myelin oligodendrocyte glycoprotein (MOG) in the subject.

Optionally, the level of expression of at least one of vimentin, ionized calcium binding adaptor molecule 1 (lba1), and interleukin-6 (IL-6) in the subject greater than the level of expression of at least one of myelin basic protein (MBP), and myelin oligodendrocyte glycoprotein (MOG) in the subject indicates a leukodystrophy in the subject.

Further optionally, the level of expression of vimentin, ionized calcium binding adaptor molecule 1 (lba1), and interleukin-6 (IL-6) in the subject greater than the level of expression of myelin basic protein (MBP), and myelin oligodendrocyte glycoprotein (MOG) in the subject indicates a leukodystrophy in the subject.

Still further optionally, the level of expression of vimentin, and ionized calcium binding adaptor molecule 1 (lba1) in the subject greater than the level of expression of myelin basic protein (MBP), and myelin oligodendrocyte glycoprotein (MOG) in the subject indicates a leukodystrophy in the subject.

### Brief description of the drawings

The present invention will now be described with reference to the accompanying drawings in which:

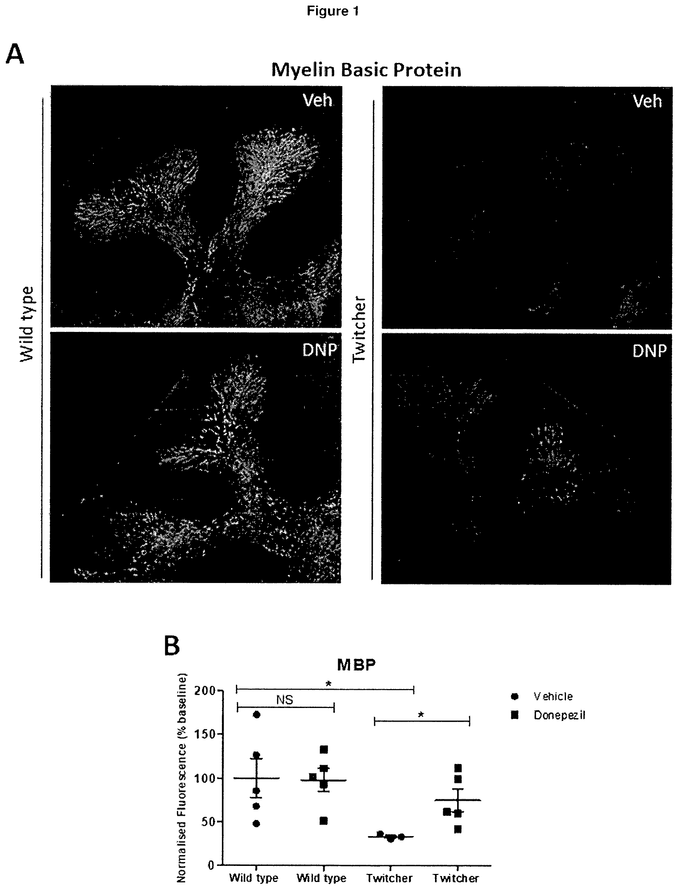

Figure 1 illustrates donepezil increases levels of MBP in twitcher mice, wherein (A) representative confocal images display MBP immunostaining under the different treatment conditions (vehicle, Veh; donepezil, DNP), wherein the images were captured at 10x magnification, focusing on cerebellar lobes (scale bar, 200 micrometres (µm)); wherein (B) graphs illustrate the donepezil-treatment effect on MBP normalized fluorescence (% baseline), wherein differences between the treatment groups were statistically analyzed applying one-way ANOVA followed by a Bonferroni correction for multiple comparisons, as well as Mann-Whitney nonparametric test between all group pairs (NS, not significant, *p<0.05, n=3-5 animals); and wherein graphical data are represented as the mean ± SEM;

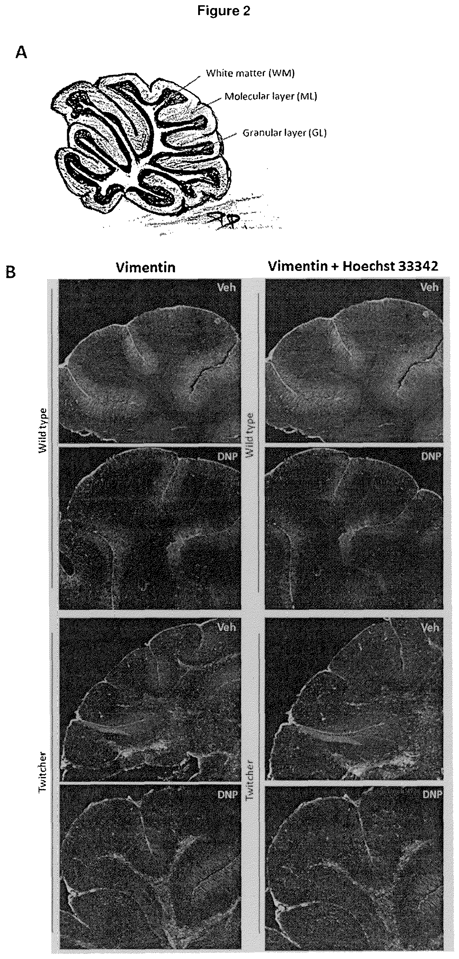

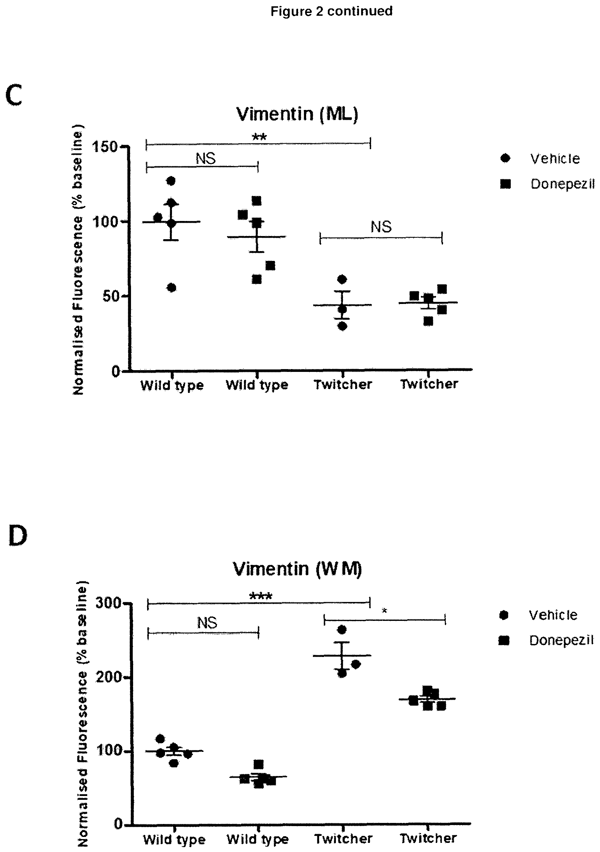

Figure 2 illustrates the effects of donepezil on astrocytes in twitcher mice, wherein (A) a sagittal mouse cerebellum scheme illustrate the different anatomical layers; molecular layer (ML), granular layer (GL), white matter (WM); wherein (B) representative confocal images display Vimentin immunostaining, alone and with Hoechst 33342 counterstain under the different treatment conditions (vehicle, Veh; donepezil, DNP), wherein the images were captured at 10x magnification, focusing on cerebellar lobes (scale bar, 200 micrometres (µm)); wherein graphs illustrate the donepezil-treatment effect on Vimentin normalized fluorescence (% baseline) at the (C) Bergmann glial cells of molecular layer (ML) and (D) fibrous astrocytes of white matter (WM), wherein differences between the treatment groups were statistically analyzed applying one-way ANOVA followed by a Bonferroni correction for multiple comparisons, as well as Mann-Whitney nonparametric test between all group pairs (NS, not significant, *p<0.05, **p<0.01, ***p<0.001, n=3-5 animals), and wherein graphical data are represented as the mean ± SEM;

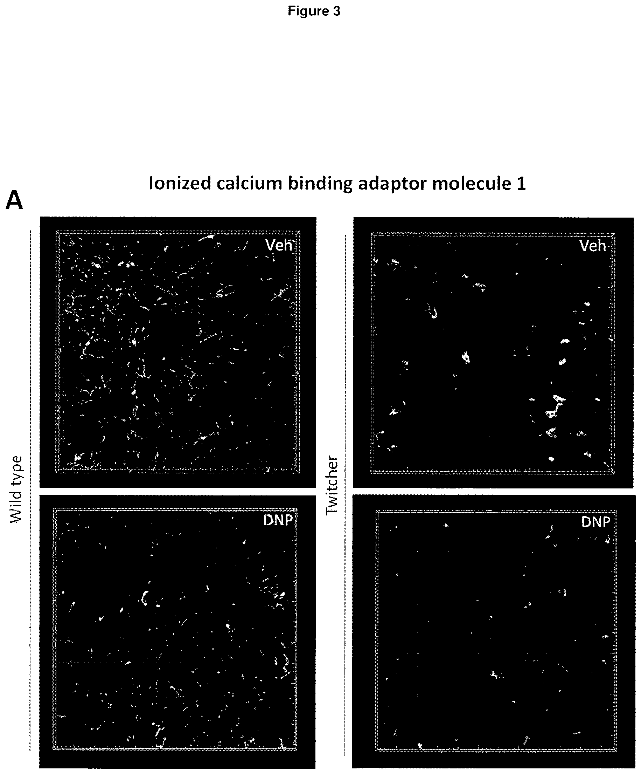

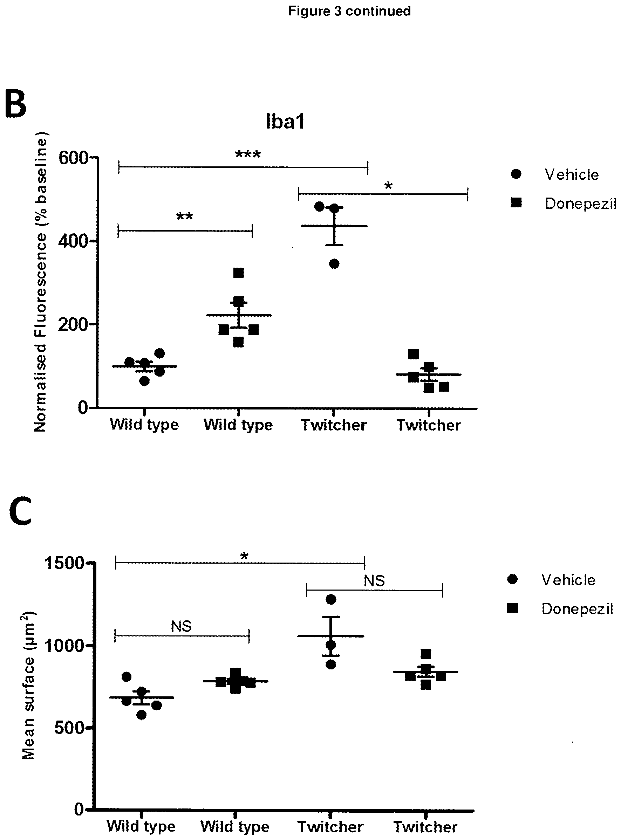

Figure 3 illustrates the evaluation of microglia in wild type and twitcher mice treated with donepezil, wherein (A) representative images created using IMARIS software display Iba1 immunostaining under the different treatment conditions (vehicle, Veh; donepezil, DNP), wherein the images were captured at 20x magnification, focusing on cerebellar lobes (scale bar, 100 micrometres (µm)); wherein (B) a graph illustrates the donepezil-treatment effect on lba1 normalized fluorescence (% baseline); wherein (C) a graph illustrates the Iba1-positive areas expressed in square micrometers, wherein differences between the treatment groups were statistically analyzed applying one-way ANOVA followed by a Bonferroni correction for multiple comparisons, as well as Mann-Whitney nonparametric test between all group pairs (NS, not significant, *p<0.05, **p<0.01, ***p<0.001, n=3-5 animals); and wherein graphical data are represented as the mean ± SEM;

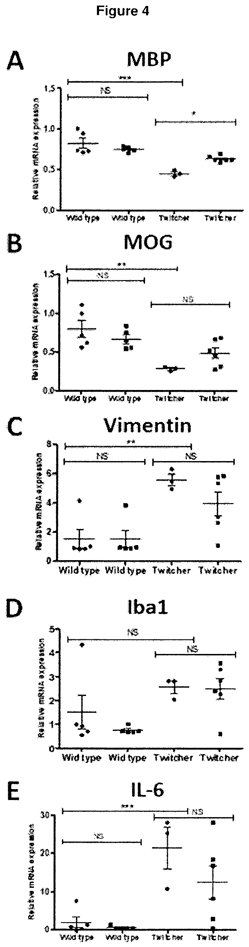

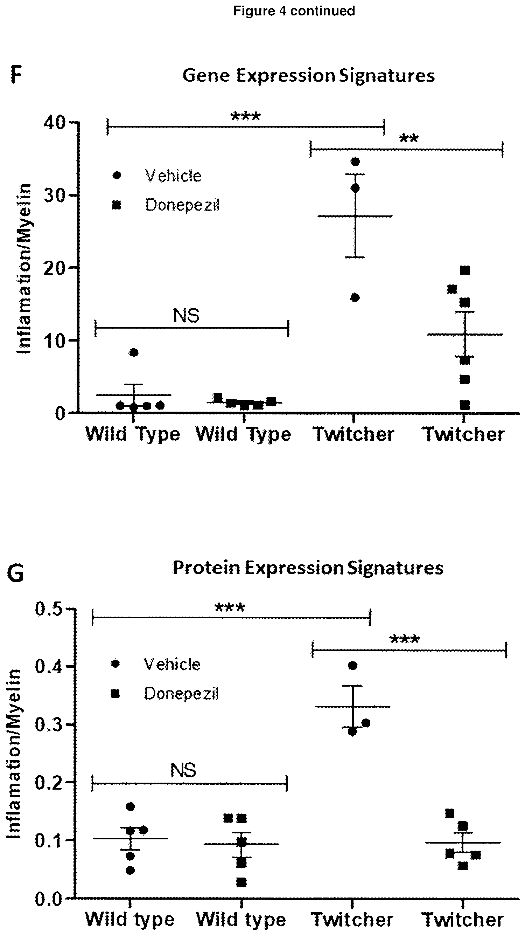

Figure 4 illustrates the evaluation of donepezil effect on a combined myelin, glial, inflammatory gene expression signature, wherein graphs illustrate the donepezil-treatment effect on relative mRNA expression of (A) MBP, (B) MOG, (C) Vimentin, (D) Iba1, and (E) IL-6 genes using RT-qPCR analysis; wherein (F) a graph shows algorithm analysis of gene-expression-signature using RT-qPCR data in which an average of expression values for glia and cytokine inflammatory markers (Vimentin, Iba1 and IL-6) was divided by an average of expression values for myelin markers (MBP and MOG), for each individual animal; wherein (G) a graph shows algorithm analysis of protein-expression-signature using immunocytochemistry data in which an average of expression values for glia cell markers (Vimentin and lba1) was divided by an average of expression values for myelin markers (MBP and MOG), for each individual animal, wherein differences between the treatment groups were statistically analyzed applying one-way ANOVA followed by a Bonferroni correction for multiple comparisons, as well as Mann-Whitney nonparametric test between all group pairs (NS, not significant, *p<0.05, **p<0.01, ***p<0.001, n=3-5 animals); and wherein graphical data are represented as the mean ± SEM; and

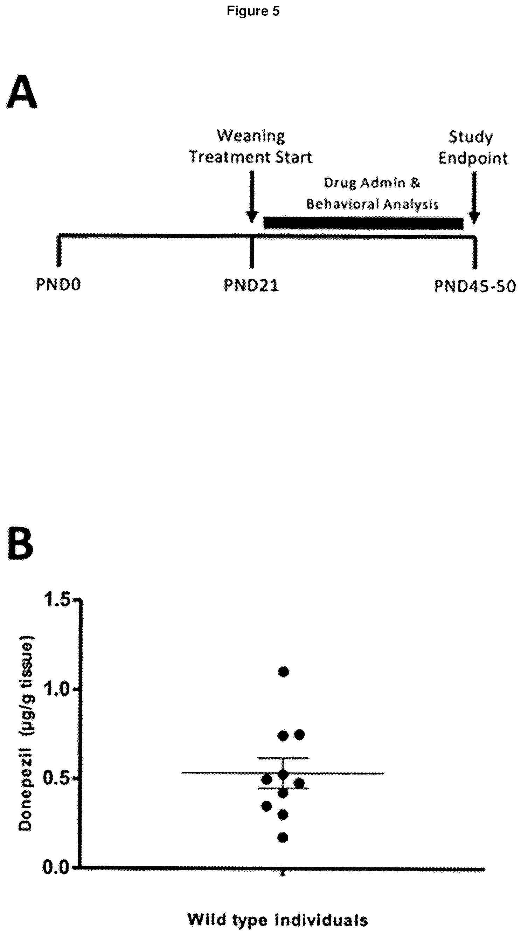

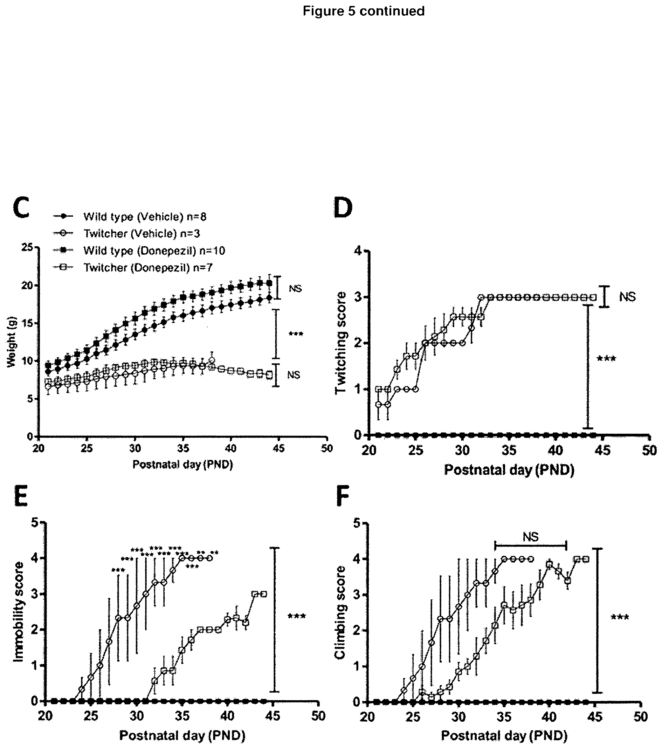

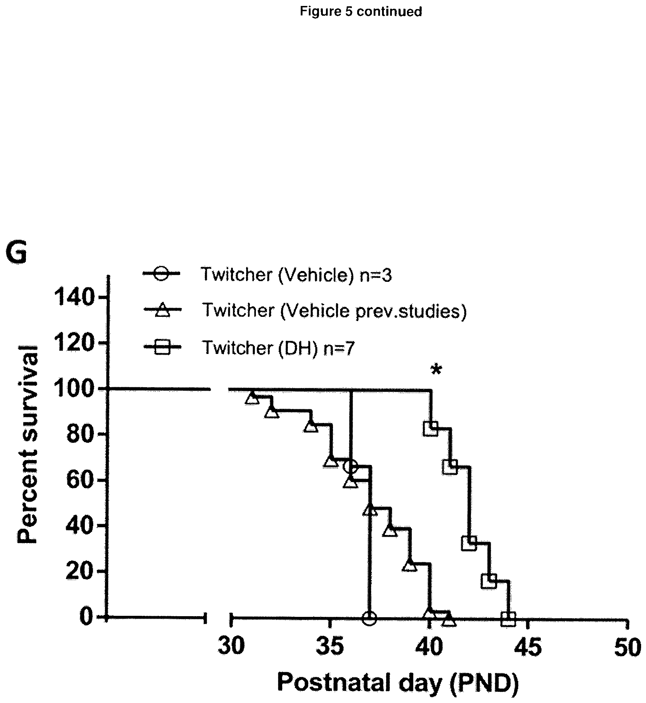

Figure 5 illustrates donepezil enhances the lifespan of twitcher mice; wherein (A) illustrates schematic representation of the study design, wherein donepezil treatment commenced at P21 at a concentration with daily behavior monitoring; wherein (B) illustrates brain concentration levels of donepezil in wild type animals, after 44 days of daily administration (10 mg/kg) via the drinking water; wherein (C) illustrates body weight of wild type and twitcher mice treated with vehicle or donepezil (two-way ANOVA followed by a Bonferroni adjustment for multiple comparisons, (***p<0.001, n=3-8 animals); wherein behavioral analysis of donepezil-treatment effect is illustrated on (D) twitching, (E) immobility, and (F) climbing score (two-way ANOVA followed by a Bonferroni adjustment for multiple comparisons (NS, not significant, **p<0.01, ***p<0.001, n=3-8 animals); wherein (G) the Kaplan-Meier survival curve depicts the lifespan increase of donepezil-treated versus untreated twitcher mice, wherein the survival curve (blue line) of untreated twitcher mice from previous studies (Bechet S, O'Sullivan SA, Yssel J, Fagan SG, Dev KK. Fingolimod Rescues Demyelination in a Mouse Model of Krabbe's Disease. J Neurosci. 2020;40(15):3104-3118. doi: 10.1523/JNEUROSCI.2346-19.2020; and Sharma K, Dev KK. The Effects of Antipsychotics in Experimental Models of Krabbe Disease. Biomedicines 2023, 11, 1313. doi: 10.3390/biomedicines11051313) were included in the graph for comparison, wherein a log-rank Mantel-Cox test reveals that the donepezil-treated twitcher group (n=7) was significantly different from vehicle-treated groups (current study, n=3, *p<0.05; previous studies, n=33, *p<0.05).

### Examples

The present invention will now be described with reference to the following non-limiting examples:

### Materials and Methods

### Animals and housing conditions

A breeding colony of heterozygous twitcher mice was established in a pathogen-free environment in the Comparative Medicine Unit at Trinity College Dublin using mice obtained from the Jackson Laboratory and maintained on a C57BL/6J genetic background (C57BL/6J-twi with C57BL/6J-twi). Homozygous and heterozygous animals were identified using genotyping procedure. Heterozygous animals were used only for breeding purposes and were not otherwise used in the study. The mice used in the study were housed in ventilated cages (Techniplast, IT-Varese) under specific pathogen-free conditions and constant environmental conditions (12:12 h light dark cycle, temperature 22 ± 2 deg. °C, relative humidity 45 ± 10 %). The mice had free access to food and tap water. All mice in the facility were screened regularly according to a health-monitoring program, complying with the Federation of European Laboratory Animal Science Associations' recommendations. The experimental protocol of the study was approved by the Health Products Regulatory Authority (HPRA, project authorization number. AE19136 /P123).

### Experimental protocol

Donepezil hydrochloride (MW: 379.50 g/mol, Cipla Ltd., India) was administered to both male and female wild-type and homozygous twitcher mice via drinking water (concentration, 20 microgram (µg)/mL), which would result in a calculated final dose of 10 mg/kg/d, considering a daily water consumption equal to 3 mL for each mouse (Sheridan GK, Dev KK. Targeting S1P receptors in experimental autoimmune encephalomyelitis in mice improves early deficits in locomotor activity and increases ultrasonic vocalisations. Sci Rep. 2014;4:5051. doi: 10.1038/srep05051). Drinking water is considered as vehicle of the administered drug. Wild type and twitcher mice were used in the study, after being divided into two subgroups as follows: vehicle (H₂O) group (n=8 wild type and n=3 twitcher mice) and donepezil group (n=10 wild type and n=7 twitcher mice). The rationale for using a low number of vehicle (H₂O) treated twicher mice (n=3) was primarily based on animal ethics and considered in line with 3R principles. In this case, as a benchmark, two previously published separate studies were used showing the effect of vehicle (H₂O) administration in twitcher mice. Thus, the twitcher vehicle group used in this study was analyzed in comparison to previous data of twitcher mice treated with water following the same protocol (n=33, Béchet et al., 2020 and n=8, Sharma and Dev 2023). Dose selection for oral administration was based on literature pharmacokinetic data (Shin CY, Kim HS, Cha KH, Won DH, Lee JY, Jang SW, Sohn UD. The Effects of Donepezil, an Acetylcholinesterase Inhibitor, on Impaired Learning and Memory in Rodents. Biomol Ther. 2018;26(3), 274-281. doi: 10.4062/biomolther.2017.189).

### Behavioral analysis

The behavioral analysis of wild type and twitcher mice was adapted from the previous studies (Bechet et al. 2020; Sharma and Dev 2023), using a modified version of the scoring systems published by Wicks SE, Londot H, Zhang B, et al. Effect of intrastriatal mesenchymal stromal cell injection on progression of a murine model of Krabbe disease. Behav Brain Res. 2011;225(2):415-425. doi: 10.1016/j.bbr.2011.07.051 and Beeton C, Garcia A, Chandy KG. Induction and clinical scoring of chronic-relapsing experimental autoimmune encephalomyelitis. J Vis Exp. 2007;(5):224. doi: 10.3791/224 to assess the twitching frequency and mobility, respectively. Briefly, all animals were subjected to a behavioral analysis beginning at 21 d of age [postnatal day 21 (P21)], while the donepezil treatment started on the 25^{th} day. Behavioral observations were taken daily up to three times per day, and body weight was measured at each behavior testing time point. Very mild and fine twitching, similar to postweaning symptoms, were scored as score 1. Mild, intermittent fine twitching was given a score of 2, while constant fine twitching corresponded to a score of 3. Constant moderate twitching of body and head was given a score of 4 and was followed by euthanasia if the mouse had a score of 4, during four independent observations. Constant and severe trembling and uncontrollable twitching was the highest score, followed immediately by euthanasia. The locomotor deficits arising due to demyelination were quantified considering that the minimum score of 1 indicates the absence of the disease, while the maximum score of 4. Fine and easy climbing with long duration was scored with 0, while late fine climbing with 1. Late and short-duration climbing had a score of 2, while the grasping and falling was scored with 3. The maximum climbing score was 4 and it was equivalent to the inability to climb. The genotype of the animals was performed on the 15^{th} postnatal day (P15). The water bottles were filled by the experimenter with 250 mL of water, where the weighted amount of the drug was dissolved, and thus no blinding was included at the level of drug treatment. All data were randomized, and analysis was performed with data blinded to both genotype and drug treatment.

### High-performance liquid chromatography (HPLC) analysis

Donepezil extraction from brain tissue has been described and validated by Papakyriakopoulou et al. (Papakyriakopoulou P, Rekkas DM, Colombo G, Valsami G. Development and In Vitro-Ex Vivo Evaluation of Novel Polymeric Nasal Donepezil Films for Potential Use in Alzhei'er's Disease Using Experimental Design. Pharmaceutics. 2022; 14(8):1742. doi: 10.3390/pharmaceutics14081742), using quercetin as internal standard (ISTD). Shortly, on the day of analysis, each brain sample was homogenized prior to analysis with a T10 ULTRA-TURRAX^{®} (IKA Werke, DE-Staufen im Breisgau) in presence of water for injection (WFI) (tissue:WFI ratio 1:1 w/w), and then 25 microlitres (µL) of homogenate tissue was vortex-mixed with 50 microlitres (µL) of ISTD (quercetin 2 pg/mL in methanol) and 25 microlitres (µL) of mobile phase. The mixture was centrifuged and 30 microlitres (µL) of the supernatant was received and directly injected onto the HPLC system for donepezil quantification. The HPLC system is composed by a LC-20AD Quaternary Gradient Pump with degasser, with an SIL-HT auto-sampler and a photo-diode array detector SPD-M20A. Analysis was carried out on an analytical reverse phase MZ Analysentechnik Nucleosil 100-5 C18 column (125×4.6 mm, 5 micrometre (µm) particle size) connected to a precolumn C-18 (12.5×4.6 mm, 5 micrometre (µm) particle size, MZ Analysentechnik) of the same type. Mobile phase consisted of phosphate buffer: methanol: acetonitrile (50:40:10) and adjusted to pH 2.8 with orthophosphoric acid (80%), in isocratic mode with flow rate 0.8 mL/min. The LC analysis time was 10 min. The injection volume was 30 microlitres (µL) and the retention time of donepezil and ISTD was 4.5 and 8 min, respectively. Detection of donepezil and Que was performed at 268 and 369, respectively, and the calibration curve samples ranged from 0.05 to 3 microgram (µg)/mL of donepezil.

### Processing of tissue for immunohistochemistry (IHC)

Mice were euthanized by placing in a COz chamber and then transcardially perfused with 20 mL of ice-cold PBS, pH 7.4 (flow: 7 mL/min), using a peristaltic pump (Watson-Marlow, Falmouth, Cornwall, UK) to remove the residual blood. Once perfusion was completed, animals were decapitated, and brains were removed and split into two hemispheres. One hemisphere was further separated into cerebellum and cortex, and then stored at 80°C until processed for quantitative reverse transcription PCR (RT-qPCR) and HPLC analysis. The remaining hemisphere was postfixed in 4% paraformaldehyde overnight (4 deg.°C) before being cryoprotected in 30% sucrose solution (4 deg.°C). Brains were then snap frozen on dry ice and embedded in optimal cutting temperature compound (OCT; catalog #361603E, VWR). Parasagittal cerebellar cryosections of 12 micrometre (µm) thickness were cut using the Leica CM1850 Cryostat and processed for IHC.

### Histology and immunofluorescence

Cerebellar cryosections were equilibrated to room temperature for 2h, and then rehydrated with 100 microlitres (µL) of PBS on each section. At all the steps of the procedure, the same volume was applied on every section. Sections were permeabilized using 0.2% Triton X-100 (Tx; catalog #T9284, Sigma-Aldrich) in phosphate buffer saline (PBS; catalog #18912-014, gibco) to reduce nonspecific binding and were blocked with 10% bovine serum albumin (BSA; catalog #A3294-100G, Sigma-Aldrich) for 2 h at room temperature. Primary antibody incubations were conducted overnight at 4 deg.°C in PBS containing 2% BSA and 0.1% Tx. Primary antibodies used were anti-myelin basic protein (MBP; 1:500 dilution; catalog #M3821-100UG, Sigma Aldrich), IbA1 Polyclonal Antibody (1:500 dilution; catalog #PA5-27436, Invitrogen), and anti-vimentin (1:500 dilution; catalog #sc-373717, Santa Cruz Biotechnology). Secondary antibody incubations were performed overnight at 4 deg.°C in PBS supplemented with 2% BSA and 0.1% Tx. The secondary antibodies used in the study were as follows: Invitrogen goat anti-mouse Alexa Fluor 488 (1:500; A11001, Thermo Fisher Scientific), Invitrogen goat anti-chicken Alexa Fluor 633 (1:500; A21103, Thermo Fisher Scientific), and Invitrogen anti-rabbit Alexa Fluor 488 (1:500; A11008, Thermo Fisher Scientific). A counterstain with Hoechst 33342 (1:10,000; catalog #62249, Thermo Fisher Scientific) labeling the nucleus was performed at the end of the immunofluorescence protocol. Slices were mounted using the Invitrogen SlowFade^{™} Gold antifade reagent (S36936, Thermo Fisher Scientific), coverslipped on microscope slides and stored at 4 deg.°C, in the dark, before being imaged.

### Light and fluorescence microscopy

Data acquisition and quantification were similar to previous studies (Sheridan and Dev, 2014). Confocal images captured for quantitative measurement of immunofluorescent staining were 12 bit.lif files of 2048 × 2048-pixel resolution and were randomly acquired throughout the cerebellum. They were captured using a Leica Sp8 scanning confocal microscope with 10× and 20× objective. With four cerebellar slices per slide, 10-12 images were taken per slide to cover most of the total area of the cerebellum. Five slides were used per treatment group, making a minimum of 50 images analyzed per treatment group. Image acquisition settings were kept the same across different treatments per experiment. Image analysis was conducted using the software Imaged (https://imagej.nih.gov/ij/). Regions of interest were manually selected on each image, and fluorescence intensity was averaged. Fluorescence intensity results are shown in normalized fluorescence (% baseline) units. For Iba1 quantification, in addition to fluorescence intensity images, we used z-stacks to compute 3D models of Iba1-positive microglia with Imaris software (Bitplane). The surface of those 3D models was then used to identify the area and volume taken up by microglia, with the hypothesis that the amoeboid and reactive state of microglia in twitcher mice will be reflected in higher volume measurements.

### Reverse transcription quantitative PCR (RT-qPCR) analysis

Total RNAs were isolated from cerebellum tissues using the Nucleospin^{®} total RNA isolation kit (Macherey-Nagel, Germany). The concentrations of isolated RNA from each sample were assessed using a NanoDrop 2000 Spectrophotometer (Thermo Scientific, USA). 10 µL of the isolated RNA were equalized to 20 microlitres (µL) using RNase-free water. Then, the cDNA was synthesized by reverse transcription using the High-Capacity cDNA manufacture kit (Applied Biosystems^{™}, Thermo Fisher Scientific, USA). Equal volumes of the isolated RNA and cDNA mastermix were mixed, centrifuged, and loaded in the thermal cycler (T100^{™}, BIO-RAD, USA). The cDNA was diluted 1:2 with RNase-free water and stored at -20 deg.°C prior to qPCR analysis. The manufactured cDNA was amplified using multi-target qPCR. cDNA and PCR mastermix, containing the target and endogenous primers, as well as the Taqman mastermix, were loaded on a 96-well PCR fast plate (Thermo Fisher Scientific, USA) in an ice block. The plate was centrifuged (1000 rpm, 1 min) to ensure adequate mixing and then inserted into the Step One Real Time PCR System, using GAPDH (Hs02786624_g1, VIC) as endogenous control. The analysis was performed on specific gene-targets, using myelin markers (MBP, myelin oligodendrocyte glycoprotein: MOG), astrocyte (Vimentin) and microglia (Iba1) markers, as well as the proinflammatory (IL-6) gene. Gene expression data were analyzed using the Livak and Schmittgen (2001) ΔΔCt method, to produce a relative quantity (RQ) value of target gene expression relative to GAPDH expression as per formula in equations 1-3: (1) ΔCₜ=Cₜ target- Ct endogenous; (2) ΔΔCₜ= ΔCₜ sample- ΔCₜ control sample; (3) RQ=2-ΔΔCₜ.

### Inflammatory gene and protein expression signatures

Inflammatory gene and protein expression signatures were generated for each animal using the 5 genes (MBP, MOG, Vimentin, lba-1, and IL-6). For the genes/proteins that are positively associated with myelin (e.g. MBP and MOG), the original data were used. Conversely, for the genes/proteins negatively associated with myelin (e.g. glia reactivity and inflammation), the inverse data were used. Subsequently, the inverted data of Vimentin, lba-1, and IL-6 genes/proteins were averaged and divided by the average value of MBP and MOG genes/proteins original data to produce the inflammation/myelin ratio. This ratio serves as an optimal signature method for the gene and protein expression of each individual animal.

### Statistical analysis

Experimental data were analyzed and graphically represented using GraphPad Prism 8.0 software package (GraphPad Software). Data normality was assessed using the Shapiro-Wilk test. The difference in the life span between twitcher and wild-type mice was tested applying the Kaplan-Meier log-rank analysis. Discrepancies in weight gain, twitching severity, and mobility impairments were analyzed using the two-way ANOVA with Bonferroni post hoc test for multiple comparisons. The minimum level of significance was set the p<0.05. Mean fluorescence intensity was measured with ImageJ software and used as an arbitrary unit of measure. Raw datasets were normalized and presented as percentages of the control group (vehicle-treated wild type animals) average. For that purpose, each arbitrary value in the experimental group was divided by the mean of the control group and multiplied by 100. The possible differences among the animal groups during the IHC and RT-qPCR analysis were assessed applying the one-way ANOVA with Bonferroni post hoc test for multiple comparisons, as well as Mann-Whitney nonparametric test between all group pairs. Graphical data are represented as the mean ± SEM.

### Example 1

### Donepezil enhances the levels of myelin in cerebellum of twitcher mice

The effects of donepezil on levels of MBP in twitcher mice was examined. The treatment of wild type animals with donepezil did not alter basal levels of MBP (100 ± 28.7 vehicle vs 98 ± 13.4 % donepezil, p = 0.841, n=5) **(****Figure 1****).** As expected, the levels of MBP were significantly reduced in vehicle-treated twitcher mice compared to wild type littermates (100 ± 28.7 wild type vs 33 ± 1.7 % twitcher, *p = 0.036, n=3-5) **(****Figure 1****).** Notably, administration of twitcher mice with donepezil significantly improved MBP expression, compared to untreated animals, where the levels of MBP in donepezil treated twitcher mice were found approaching those of wild type animals (33 ± 1.7 vehicle vs 75 ± 13.1 % donepezil, *p = 0.036, n=3-5) **(****Figure 1****).**

### Example 2

### Donepezil alters Vimentin expression in white matter layer of cerebellum in twitcher mice

KD progression is accompanied by astrocytes reactivation followed by the same event in microglia. Astrocytes in the layers of the cerebellum include the Bergmann glial cells of molecular layer (ML) and granular layer (GL) and fibrous astrocytes of white matter (WM) **(****Figure 2A****).** The type III intermediate filament astrocyte marker vimentin was employed to assess the effects of donepezil on astrocytes in twitcher mice. The fluorescence levels of vimentin in ML were decreased significantly in twitcher mice compared to wild type littermates (100 ± 14.6 vs 48 ± 10.1, **p = 0.0029, n=3-5) **(****Figure 2B**,C). These levels of vimentin in the ML were not altered by administration of donepezil either in wild type animals (100 ± 14.6 vehicle vs 89 ± 10.1 donepezil, p=0.7533, n=3-5) or twitcher animals (48 ± 10.1 vehicle vs 50 ± 4.3 donepezil, p=1.000, n=3-5) **(****Figure 2B**,C). In contrast, in twitcher mice treated with vehicle, we noted a translocation of vimentin fluorescence from the ML to GL (100 ± 7.0 wild type vs 228 ± 18.1 twitcher, ***p<0.0001, n=3-5) **(****Figure 2B**,D), which was partially reversed by treatment with donepezil (228 ± 18.1 vehicle vs 169 ± 4.4 donepezil, *p=0.0357, n=3-5) **(****Figure 2B**,D).

### Example 3

### Donepezil decreases Iba1 expression in the cerebellum of twitcher mice

The fluorescence data for ionized calcium binding adaptor molecule 1 (lba1) marker of microglia/macrophage revealed a significant effect of donepezil in wild type and twitcher treated animals **(****Figure 3****).** Namely, the drug reduced the expression of lba1 in the cerebellum of twitcher mice (437 ± 44.8 vehicle vs 82 ± 15.2 % donepezil, *p = 0.036, n=3-5) **(****Figure 3A**,B), while increasing the respective levels in wild type littermates (100 ± 14.4 vehicle vs 223 ± 30.0 % donepezil, **p = 0.0079, n=3-5) **(****Figure 3A**,B). Analysis of mean surface area of lba1 immunofluorescence showed a significant increase between vehicle treated wild-type and twitcher animals (682 ± 39.6 wild-type vs 1061 ± 117 twitcher, *p=0.0357) **(****Figure 3A**,C). Treatment with donepezil diminished these differences, where the mean surface data did not show significant variation between the vehicle and donepezil treated animals, of both wild type (682 ± 39.6 vehicle vs 786 ± 18.1 donepezil, p=0.0952, n=3-5) and twitcher groups (1061 ± 117 vehicle vs 846 ± 31.2 donepezil, p=0.0714, n=3-5) **(****Figure 3A**,C).

### Example 4

### Donepezil effect on a combined myelin, glial, inflammatory gene expression signature

KD is associated with alterations in myelin-, glial- and inflammatory-markers. In order to create a biomarker-signature of this disease model, the effects of donepezil on individual gene expression of the myelin markers (MBP, MOG) was initially examined **(****Figure 4A**,B), as well as on glia cells astrocyte (Vimentin) **(****Figure 4C****)** and microglia (Iba1) **(****Figure 4D****)** markers, and a proinflammatory cytokine (IL-6) **(****Figure 4E****),** using RT-qPCR analysis. The RT-qPCR analysis demonstrated the lower gene expression of MBP and MOG in the cerebellum of twitcher mice, while showing higher levels of vimentin, Iba1 and IL6, suggestive of a loss of myelin, enhanced glial cell reactivity and pro-inflammatory phenotypes **(**Figure 4A-E). Next, an algorithm was created to generate a gene-expression-signature in which an average of expression values for glia and cytokine inflammatory markers (Vimentin, Iba1 and IL-6) was divided by an average of expression values for myelin markers (MBP and MOG), for each individual animal **(****Figure 4F****).** These signatures showed no significant difference (p=0.2495) in wild-type mice treated with vehicle or donepezil, as expected. Importantly, an enhanced ratio of glial cell and pro-inflammatory markers versus myelin markers was observed in twitcher mice compared to wild-type mice (p=0.0001). Moreover, the administration of twitcher mice with donepezil showed a significant decrease (p=0.0016) in this gene expression signature compared to vehicle treated twitcher mice **(****Figure 4F****).** A similar algorithm was also generated for immunocytochemistry analysis, where an average of expression values for glia cell markers (Vimentin and Iba1) was divided by an average of expression values for myelin markers (MBP and MOG), for each individual animal **(****Figure 4G****).** These protein expression signatures agreed with gene expression signature data, showing enhanced glia cell reactivity versus myelin levels in twitcher mice (p<0.0001), that was significantly attenuated in twitcher mice treated with donepezil (p<0.0001) **(****Figure 4G****).**

### Example 5

### Donepezil increases the lifespan of twitcher mice

Administration of drug (donepezil) and behavioral analysis was initiated upon weaning of animals at PND21. The immunocytochemistry and RT-qPCR analysis indicated in the data above was conducted at the study endpoint **(****Figure 5A****).** Drug brain levels of donepezil were measured in wild type littermates treated with 10 mg/kg donepezil via drinking water for 44 days using HPLC analysis **(****Figure 5A****).** The concentration values correspond to the brain drug levels on the day of sacrifice (P44) and they are normally distributed around the mean value (p= 0.6753). Outlier detection occurred by applying the interquartile range (IQR) using a step of 1.5 × IQR. No outliers were detected (p> 0.05, for all the tested individuals). The concentration of donepezil in the brains of twitcher mice were not detectable when sacrificed, in line with the daily water consumption of mice being reduced the end-stage of life and daily hydration mostly via a drug-free gel food.

Donepezil oral administration in twitcher mice via the drinking water from P25 onward showed an increased trend in the body weight from the P27 to P32 compared to vehicle treated twitcher animals, which was not different between these two twitcher groups from P32 onwards to end stage of life **(****Figure 5C****).** Twitching scoring was progressive with disease in twitcher mice and did not reveal any significant difference between the donepezil treated and vehicle treated animals **(****Figure 5D**), where both groups plateaued at P32-P33, until the endpoint of the study. In the case of immobility score, a significant positive effect of donepezil treatment is noted from the P28 onwards, where the drug delayed appearance of immobility by approximately 8 days as well as decreased severity of this phenotype **(****Figure 5E****).** We also monitored the natural climbing ability of mice on the cage rack and noted that twitcher mice showed decreased climbing score compared to wild-type littermates **(****Figure 5F****).** In this case, we scored fine and easy climbing with long duration at 0 (as seen in wildtype mice and twitcher mice until 20-25 days of age), while late fine climbing was scored with 1, late and short-duration climbing had a score of 2, grasping and falling was scored with 3 and the inability of climbing was scored at 4. The loss of climbing behavior was delayed in donepezil-treated twitcher mice compared to vehicle-treated **(****Figure 5E****).** These overall mobility assessments constitute an efficacy of donepezil in the management of behavioral symptoms in twitcher mice. The effect of donepezil on lifespan of twitcher animals was also examined **(****Figure 5G****).** A decision to use of a small group size for vehicle treated twitcher animals (n=3) was taken due to (i) primarily, ethical considerations to limit use of the severe model of twitcher; (ii) also, given a recent body of evidence from previous observations that no vehicle-treated twitcher animal typically survives beyond 30-40 days (n=33) (Béchet et al., 2020; Sharma and Dev 2023), and (iii) because, therapies are being sought which promote lifespan of twitcher to beyond 40 days or more. The current findings treating twitcher mice with vehicle show as lifespan of 36 days (n=3) **(****Figure 5G****),** in agreement with previous observations (n=33) (Bechet et al., 2020; Sharma and Dev 2023). Importantly, the Kaplan-Meier survival curves demonstrate that donepezil treatment, at the dose of 10 mg/kg, increased the lifespan of twitcher mice toward 45 days, which was statistically increased compared to vehicle-treated twitcher mice in our current study and previous data **(****Figure 5G****).**

Krabbe's disease (KD) is associated with genetic mutations and subsequent loss of function of the acid hydrolase galactosylceramidase (GalC) enzyme, which plays a key role in metabolism of galactosylceramide and the toxic galactolipid galactosylsphingosine (psychosine). Strong evidence has been established about the contribution of psychosine accumulation in nervous system and its toxic effects on levels of myelin. Psychosine accumulation has been determined throughout the CNS, in the spinal cord, cerebrum, and cerebellum. A natural occurring animal model of KD, namely the twitcher mouse, displays myelin damage accompanied by axonal degeneration and the formation of globoid cells in the white matter, and is well recognized as translational model of the disease. The present invention provides a direct demonstration that donepezil rescues deficits seen at the pathological level in twitcher's mice, namely that donepezil oral treatment led to significant increase of myelin levels, restoration of astrocyte reactivity, as well as suppression of microglia activation. The current data also demonstrates that donepezil enhances mobility of twitcher animals as depicted by immobility and climbing scores, as well as promote life span in these animals.

Myelin regeneration requires the gradual replacement and renewal of OLs through a two-stages process including the differentiation of OPCs into OLs and the production of myelin sheaths around the neuronal axons. This axonal wrapping is necessary for the transmission of electrical signals along the axon. The muscarinic acetylcholine receptors (mAChRs) M1, M3, and M4 have been identified in OPCs, while all the mAChRs subtypes are reported in low levels in mature OLs. Furthermore, the co-expression of GalC and Ach transporter in a large portion of OLs population, as well as the decrease of GalC in presence of a cholinergic antagonist, suggest the involvement of ACh receptors in the differentiation of OPCs. However, only donepezil, among other AChEi such as tacrine, physostigmine, and galantamine, has been reported to display myelin-promoting effects in OPC cell culture. Sig-1Rs have been reported to play a role in regulating levels of myelin. Apart from its ability to inhibit the AChE displacing the active site of enzyme, donepezil is an agonist of Sig-1R with affinity and efficacy at low nanomolar concentrations. Positron emission tomography (PET) scan studies in both humans and rodents have also demonstrated that donepezil can bind to these receptors at therapeutic doses (5 and 10 mg produce 60 and 75% Sig-1R occupancy in humans). The donepezil EC50 value that ensures the receptor occupancy in rats was found to be 333.0 nM, corresponding to 1.07 mg/kg. Hence, a dual interaction with both AChE inhibition Sig-1R activation may be responsible for a donepezil remyelinating mechanism.

It is well-established that astrocytes and microglia play as critical role in myelin generation as regulators of migration, proliferation, and maturation stages towards the production of myelinating OLs. Both nicotinic (nAChRs) and muscarinic (mAChRs) receptors are also known to be expressed by astrocytes and microglia and response to changes in levels of ACh neurotransmitter. Thus, it is possible that enhanced levels of ACh in the brains of twitcher mice treated with donepezil may constitute a mechanism by which this drug induces the anti-inflammatory effects we observed on these glia cells. Donepezil is also a well-known agonist of Sig-1R's, where these receptors are involved in the modulation of astrocytes and microglia and regulate permeability of the blood-brain-barrier, perhaps providing another avenue for this drug to elicit its effects in twitcher mice. Donepezil is also known to attenuate amyloid-beta-induced gliosis in microglia cells and microglia activation in an AD mouse model. Pro-inflammatory signals such as IL-6 have been reported importantly enhanced in the pathological condition of KD and twitcher mice, and in agreement with the present invention, donepezil can lower neuroinflammation after the treatment. The present invention reinforces the argument of donepezil anti-inflammatory properties.

**The** observations of donepezil effects on myelin state, astrocyte and microglia reactivity and pro-inflammatory phenotypes are reflected in twitcher mouse phenotype as a 5-day life extension and improvement of mobility characteristics. The benefit of donepezil oral administration in this animal model is commensurate with the effectiveness of other marketed drugs such as fingolimod or haloperidol. The effects of these drugs are likely explained by multiorgan impairment, observed in previous works on twitcher mouse model, which unless restored, likely supersedes attempts to solely rescue dysfunction in the nervous system. The body-wide damage caused by the buildup of psychosine thereby limits the efficacy of drugs such as fingolimod, haloperidol and donepezil to show lasting improvement in twitching and mobility or increase the lifespan further. Nevertheless, the overall ameliorated phenotype and achieved increase of lifespan after donepezil treatment may not be considered negligible given the severity of this KD translational model. In conclusion, the present invention shows that donepezil has myelin-promoting properties, as well as activity against neuroinflammation and show this drug prolongs lifespan of twitcher mice.

Alzheimer's Disease (AD) is a neurodegenerative disorder characterized by cognitive decline due to the formation of neurofibrillary tangles and plaques in the brain. Current therapeutic strategies for AD target the accumulation of amyloid-beta and tau hyperphosphorylation, as well as neuroinflammation and glial cell hypertrophy. However, myelin aberrations, which are associated with AD, have also been identified as early events in the disease progression. Acetylcholinesterase inhibitors (AChEi) are commonly used for symptomatic management of AD, and are suggested to have potential disease-modifying effects, including regulating myelin state. In particular, Donepezil, an AChEi drug, promotes myelination and increases the expression of myelin-related genes. This drug also acts as an agonist for sigma-1 receptors (Sig-1R), which are implicated in demyelination diseases. Krabbe disease (KD) is a demyelinating disorder caused by mutations in the galc gene, resulting in toxic accumulation of psychosine. In the context of drug repurposing, here we demonstrate that administration of donepezil has protective effects in the twitcher mouse model of KD. The present invention provides data showing that donepezil preserves myelin and reduces glial cell reactivity in the brains of twitcher mice. Moreover, donepezil also improves behavioral phenotypes and increases lifespan in twitcher animals. The present invention shows that donepezil, with its dual activity as an AChE inhibitor and Sig-1R agonist, may hold promise as a therapeutic candidate for demyelinating diseases, including KD.

In the context of drug repurposing, the myelin-promoting properties of donepezil, along with its dual inhibitory activity against AChE and activation of Sig-1 R, render it promising candidate for the management of demyelinating diseases. The present invention shows the effect of an archetypical AchEi drug, namely donepezil, in the twitcher mouse model, a demyelinating model translatable for KD. We report that donepezil protects against the loss of myelin, dampens glial cell reactivity, slows behavioral phenotypes and increases lifespan in the twitcher mouse model of KD.

## Claims

1. An acetylcholinesterase inhibitor for use in the treatment of a leukodystrophy.

2. An acetylcholinesterase inhibitor for use according to claim 1, wherein the leukodystrophy is globoid cell leukodystrophy.

3. An acetylcholinesterase inhibitor for use according to claim 1 or 2, wherein the acetylcholinesterase inhibitor has acetylcholinesterase inhibitor and/or sigma-1 receptor (Sig-1R) agonist activity.

4. An acetylcholinesterase inhibitor for use according to any of claims 1-3, wherein the acetylcholinesterase inhibitor is a reversible acetylcholinesterase inhibitor.

5. An acetylcholinesterase inhibitor for use according to any of claims 1-4, wherein the acetylcholinesterase inhibitor is donepezil ((RS)-2-[(1-Benzyl-4-piperidyl)methyl]-5,6-dimethoxy-2,3-dihydroinden-1-one).

6. An acetylcholinesterase inhibitor for use according to any of claims 1-5, wherein the acetylcholinesterase inhibitor is donepezil hydrochloride.

7. An acetylcholinesterase inhibitor for use according to any of claims 1-6, wherein the use comprises administration of the acetylcholinesterase inhibitor orally and/or topically.

8. An acetylcholinesterase inhibitor for use according to any of claims 1-7, wherein the use comprises administration of the acetylcholinesterase inhibitor orally and/or transdermally.

9. An acetylcholinesterase inhibitor for use according to any of claims 1-8, wherein the use comprises administration of the acetylcholinesterase inhibitor at a dose of about 1-20 mg/kg.

10. An acetylcholinesterase inhibitor for use according to any of claims 1-9, wherein the use comprises administration of the acetylcholinesterase inhibitor at a dose of about 1-20 mg/kg per day.

11. An acetylcholinesterase inhibitor for use according to any of claims 1-10, wherein the use comprises administration of the acetylcholinesterase inhibitor for about 1-80 days.
